# EUROPEAN PATENT APPLICATION

(11) **EP 1 879 013 A1**
(43) Date of publication of application: **16.01.2008**
(21) Application number: 06732334.5
(22) Date of filing: 19.04.2006
(51) Int. Cl.: G01N 1/38, A61K 9/107, A61K 47/10, G01N 15/06

(54) **METHOD FOR THE DESIGN OF SELF-EMULSIFYING DRUG FORMULATIONS**

(30) Priority: 20.04.2005 JP 2005122347
(71) Applicant: CHUGAI SEIYAKU KABUSHIKI KAISHA, Tokyo, 115-8543 (JP)
(72) Inventor: SAKAI, Kenichi, Chugai Seiyaku Kabushiki Kaisya, Tokyo 1158543 (JP)
(74) Representative: Harding, Charles Thomas
(86) International application number: PCT/JP2006/308678
(87) International publication number: WO 2006/112541

(57) **Abstract**

The present invention provides an HTFS system for quickly preparing and evaluating, at low cost, an SEDDS formulation containing ingredients in a viscous liquid state or in a semi-solid or solid state. More specifically, the present invention provides a method for formulation designing of a self-emulsifying preparation, comprising the steps of: (a) diluting, in a diluent solvent, each of ingredients in a viscous liquid state or in a semi-solid or solid state to prepare each dilution containing the constituent ingredient; and (b) preparing plural test mixtures by dispensing the ingredients contained in the dilutions and ingredients other than those ingredients into plural test vessels so that the kinds and mixed amounts of the ingredients and the other ingredients added differ among the test vessels.

## Description

### Technical Field

The present invention relates to a method for designing a formulation of a self-emulsifying preparation of a poorly water-soluble drug. Specifically, the present invention relates to a method for designing a formulation of a self-emulsifying preparation, which allows the screening of the optimum formulation of the self-emulsifying preparation at a high speed.

### Background Art

In drug development, a large number of candidate compounds are evaluated and examined by pharmacological experiments to select medicinally active ingredients (hereinafter, referred to as "drugs"). Among these drugs, many drugs are poorly water-soluble. Particularly, potential drugs have been selected in recent years by high throughput screening and combinatorial chemistry based on pharmacological effects, and however, these drugs are often poorly water-soluble (Lipinski, C. A., 2000, J. Pharmacol. Toxicol. Methods 44, 235-249: Non-Patent Document 1). Poorly water-soluble compounds exhibit reduction in absorption and variations in absorption attributed to individual differences. Thus, important challenges for the development of oral preparations of poorly water-soluble compounds are to improve their water-absorbing properties and reduce variations in absorption.

A Self-Emulsifying Drug Delivery System (hereinafter, referred to as "SEDDS") comprising a drug, oil, hydrophilic and lipophilic surfactants, an absorption promoter, an auxiliary solvent, and so on has been known as a pharmaceutical technique to solve these challenges associated with absorption. SEDDS is a preparation that is prepared by uniformly mixing the above-described components in a state free from water to prepare a solution or dispersion. SEDDS, after administration, forms a fine emulsion upon dissolution and dispersion in water in the gastroentestinal tract and thereby improves absorption of a poorly water-soluble drug and variations in absorption attributed to individual differences.

In SEDDS formulation design, the formation state and stability of an emulsion discontinuously vary depending on the kind and proportion of respective components such as a drug, oil, hydrophilic and lipophilic surfactants, an absorption promoter, or an auxiliary solvent. Therefore, an approach for theoretically designing SEDDS formulations has been unknown. Therefore, the discovery of a formulation that forms an excellent, stable emulsion has required preparing a large number of formulations among which the kind and proportion of respective components variously differ and conducting screening by trial and error.

Moreover, oil and hydrophilic and lipophilic surfactants, which are main ingredients for SEDDS formulations, are generally viscous liquids or semi-solids or solids at room temperature. Therefore, formulations for examination in small amounts had to be prepared manually. Even in this case, measuring and mixing procedures are significantly difficult and a problem in throughput is presented. Moreover, drug development presented, in its early stage, the problem of a limitation in the amounts of drugs that can be used in formulation examination. Therefore, the respective components used were confined empirically, and a limited number of formulations were evaluated. Unless the respective components were confined, the number of formulations to be examined dramatically increased, resulting in the problems of prolonged examination periods, increased human efforts and amounts of drugs used, and so on.

Thus, it has been demanded to achieve a quick and efficient SEDDS formulation screening using small amounts of drugs.

In the chemical and biochemical fields, a high throughput LAS (Laboratory Automation System) technique has been prevalent by which many kinds of samples in small amounts are treated in a large-scale, high-speed manner with an automated preparation/analysis/measurement system. In LAS, the automated handling of samples with a pipette and a microplate allows quick multi-sample treatment in small amounts. It has been considered that High Throughput Formulation Screening (HTFS) for quickly screening a large number of formulations with small amounts of compounds is achieved by applying this LAS technique to SEDDS formulation screening.

However, oil and hydrophilic and lipophilic surfactants, which are ingredients for SEDDS, are generally in a viscous liquid state or in a semi-solid or solid state at room temperature. Therefore, these ingredients were difficult to dispense with a pipette and subsequently mix and could not be applied to HTFS.

On the other hand, an HTFS system for intravenous preparations has been known as a technique for preparing and evaluating the optimum formulation using surfactants for solubilizing a poorly water-soluble compound (WO 01/09391: Patent Document 1; and WO 02/43765: Patent Document 2). In SEDDS, the correct evaluation of the state of an emulsion inevitably requires uniformly mixing additives in the formulation before dispersion in water. However, in the technique described above, the surfactants are diluted with water and mixed in an aqueous solution form. Therefore, if this technique was applied to SEEDS, an emulsion was difficult to form due to the insufficient adsorption of the surfactants onto oil, resulting in the problem of incorrect evaluation of the original property of the formulation. Moreover, water had to be evaporated after mixing, also resulting in the problem of loss of quickness of preparation attributed to the slow drying speed of water.

Indexes for showing the physical properties of an emulsion include: a particle size; and the separation of an emulsion that indicates physical stability. Dynamic light scattering (hereinafter, "DLS") has been known as a method for measuring a particle size. However, DLS presented a problem in cost and quickness due to low throughput. Alternatively, a method for evaluating, with a turbidimeter, turbidity as a parameter that reflects a particle size has also been known, and however, it presented a problem in cost and quickness due to low throughput (Non-Patent Document 2: Nazzal, S., et al., 2002, Int. J. Pharm. 235, 247-265). Visual observation of the appearance is required to determine separation of an emulsion (Non-Patent Document 3: Kawakami., K., et al., 2002, J. Controlled Release 81, 65-74). However, this technique exhibited variations in determination among observers and presented a problem in cost and quickness due to its low throughput.

In light of these situations, it has been demanded to develop an HTFS system for quickly preparing and evaluating, at low cost, an SEDDS formulation containing ingredients in a viscous liquid state or in a semi-solid or solid state.

### Disclosure of the Invention

The present inventor has conducted diligent studies for achieving the object and has developed a system capable of continuously performing the dispensing, mixing, and measurement of small amounts of samples at a high speed in the evaluation and examination of a large number of SEDDS formulations. Specifically, the present invention provides a method for designing a formulation of a self-emulsifying preparation shown below.
(1) A method for formulation designing of a self-emulsifying preparation of a poorly water-soluble drug, comprising the steps of: (a) dissolving, in a diluent solvent, each of the poorly water-soluble drug and ingredients of the preparation in a viscous liquid state or in a semi-solid or solid state at room temperature to prepare each dilution containing the ingredient; and (b) if additional ingredients of the preparation that are dispensable with a dispensing apparatus at room temperature are required, using each of the dispensable ingredients as it is without dissolution in a diluent solvent or dissolving each of the dispensable ingredients in a diluent solvent to prepare each dilution containing the dispensable ingredient, and preparing plural test mixtures by dispensing all these ingredients into plural test vessels so that the kinds and mixed amounts of the ingredients added differ among the test vessels.
(2) The method according to (1), further comprising the steps of: (c) removing the diluent solvent from the plural test mixtures obtained; (d) adding water or a test solution to the plural test mixtures obtained; and (e) evaluating the properties of emulsions formed from the plural test mixtures thus obtained.
(3) The method according to (1) or (2), wherein the diluent solvent is alcohols.
(4) The method according to (3), wherein the diluent solvent is an alcohol having 2 to 5 carbon atoms.
(5) The method according to (4), wherein the diluent solvent is ethanol, n-propanol, isopropanol, or a mixture thereof.
(6) The method according to any of (2) to (5), wherein the step (e) of evaluating the properties of emulsions formed from the test mixtures comprises the step of evaluating the particle sizes of the emulsions by turbidity measurement.
(7) The method according to any of (2) to (6), wherein the step (e) of evaluating the properties of emulsions formed from the test mixtures comprises the step of evaluating the separation stability of the emulsions by the measurement of a turbidity change between before and after storage treatment.
(8) The method according to any of (2) to (7), wherein the step (e) of evaluating the properties of emulsions formed from the test mixtures comprises the step of evaluating the separation stability of the emulsions by the measurement of a turbidity change between before and after centrifugation treatment.
(9) The method according to any of (2) to (8), further comprising the step of determining the optimum formulation of the self-emulsifying preparation on the basis of evaluation results obtained in the step of evaluating the properties of emulsions formed from the text mixtures.
   The present invention further provides a method for evaluating an emulsion shown below.
(10) A method for evaluating the property of an emulsion, comprising the step of evaluating the particle size of the emulsion by turbidity measurement.
(11) A method for evaluating the property of an emulsion, comprising the step of evaluating the separation stability of the emulsion by the measurement of a turbidity change between before and after storage treatment.
(12) A method for evaluating the property of an emulsion, comprising the step of evaluating the separation stability of the emulsion by the measurement of a turbidity change between before and after centrifugation treatment.

SEDDS formulation design requires, due to the discontinuously varying formation state and stability of an emulsion, screening by trial and error in such a way that a large number of formulations are prepared and evaluated. However, the present invention allows HTFS (High Throughput Formulation Screening) exploiting the convenience and quickness of LAS (Laboratory Automation System) for such formulation design. Thus, the preferable aspect of the present invention can provide a method for quickly preparing and evaluating many formulations of preparations at low cost with small amounts of drugs used. Moreover, the method for evaluating an emulsion according to the present invention has the advantage of being capable of evaluating an emulsion more quickly and accurately than conventional evaluation methods.

### Brief Description of the Drawings

Figure 1 shows measurement results of turbidities of obtained samples;
Figure 2 shows measurement results of a turbidity change after centrifugation of obtained samples; and
Figure 3 shows a turbidity change after storage at 25°C for 48 hours of obtained samples.

### Best Mode for Carrying Out the Invention

First, the present invention provides a method for formulation designing of a self-emulsifying preparation of a poorly water-soluble drug, comprising the steps of:
(a) dissolving, in a diluent solvent, each of the poorly water-soluble drug and ingredients of the preparation in a viscous liquid state or in a semi-solid or solid state at room temperature to prepare each dilution containing the ingredient; and (b) if additional ingredients of the preparation that are dispensable with a dispensing apparatus at room temperature are required, using each of the dispensable ingredients as it is without dissolution in a diluent solvent or dissolving each of the dispensable ingredients in a diluent solvent to prepare each dilution containing the dispensable ingredient, and preparing plural test mixtures by dispensing all these ingredients into plural test vessels so that the kinds and mixed amounts of the ingredients added differ among the test vessels. The preferable aspect of the present invention further comprises the steps of: (c) removing the diluent solvent from the plural test mixtures obtained; (d) adding water to the plural test mixtures obtained; and (e) evaluating the properties of emulsions formed from the plural test mixtures thus obtained.

In the present specification, the "self-emulsifying preparation" refers to a Self-Emulsifying Drug Delivery System (SEDDS) comprising a poorly water-soluble drug, oil, hydrophilic and lipophilic surfactants, an absorption promoter, an auxiliary solvent, and so on (see e.g., Gursoy, N. R., et al., 2004, Biomedicine & pharmacotherapy 58, 173-182). In the method of the present invention, a series of the steps (a) to (e) can be automated. Thus, the method of the present invention has the advantage of being capable of optimizing a formulation of a self-emulsifying preparation at a higher speed with higher accuracy than conventional methods.

Hereinafter, each step of the present invention will be described.

### 1. Step (a) of preparing dilution

In the step (a) of preparing dilutions, each of ingredients in a viscous liquid state or in a semi-solid or solid state at room temperature is dissolved in a diluent solvent to prepare each dilution containing the constituent ingredient. In this context, the "ingredients in a viscous liquid state or in a semi-solid or solid state at room temperature" are ingredients to be added to the self-emulsifying preparation and refer to substances that cannot be dispensed with a dispensing apparatus (tool) such as a pipette without dilution with a diluent solvent. Alternatively, the "ingredients dispensable with a dispensing apparatus at room temperature" are ingredients to be added to the self-emulsifying preparation and refer to substances that can be dispensed with a dispensing apparatus without particular dilution with a diluent solvent.

Examples of the ingredients to be added to the self-emulsifying preparation include a poorly water-soluble drug (active ingredient), oil, lipophilic and hydrophilic surfactants, an absorption promoter, and an auxiliary solvent. In the present invention, when any one or more of the active ingredient, the oil, and the lipophilic and hydrophilic surfactants, which are main ingredients for SEDDS, are in a viscous liquid state or in a semi-solid or solid state at room temperature, such ingredients are diluted with a diluent solvent such as lower alcohols to thereby allow for dispensing procedures and, by extension, for automation. Thus, the ingredient diluted with a diluent solvent is not limited to one of the ingredients described above, and two or more or all of ingredients may be diluted. In the present specification, "dilution" refers to dissolution or reduction in viscosity to the extent allowing for dispensing, for example, with a dispensing apparatus (tool) used. Thus, those ingredients do not have to be dissolved completely in the diluent solvents as long as they are dispensable with a pipette or the like. In the preparation of the dilutions, the ingredients to be diluted and/or the diluent solvent can be heated, if necessary.

The "diluent solvent" used in the present invention is not particularly limited as long as it can solubilize the ingredients (SEDDS ingredients) in a viscous liquid state or in a semi-solid or solid state to the extent allowing for dispensing. Examples of such a diluent solvent include alcohols (ethanol, n-propanol, isopropanol, and benzyl alcohol), dimethyl sulfoxide, N,N-dimethylacetamide, tetrahydrofuran, dioxane, dichloromethane, chloroform, and mixtures thereof. A preferable diluent solvent is a solvent that does not interfere with the mixing of oil with surfactants and does not cause the problem of poor emulsion formation seen with water as a solvent.

In light of such properties, a preferable diluent solvent is a lower alcohol having a polarity smaller than that of water. In this context, the "lower alcohol" refers to a linear or branched alcohol having 2 to 5 carbon atoms, and examples thereof include methanol, ethanol, n-propanol, isopropanol, n-butanol, 2-butanol, 2-methyl-1-propanol, and 2-methyl-2-propanol. Among these lower alcohols, more preferable solvents are methanol, ethanol, n-propanol, isopropanol, and mixtures thereof. They are excellent not only in the solubility of additives used in SEDDS but also in solvent removal after preparation because of low boiling points. Moreover, the diluent solvent is removed by drying, if necessary, and the lower alcohols have the advantage of being capable of being removed by drying more easily and quickly than water.

In the present invention, although the diluent solvent such as lower alcohols is usually removed by evaporation, it slightly remains in some cases. Thus, a formulation in which the lower alcohol remains might be selected as a result of screening. However, even if the residual diluent solvent is judged as a necessary component, ethanol when used as such a diluent solvent can be incorporated easily as a highly safe additive into the actual preparation and is therefore less likely to cause a big problem in development. Thus, among C₂ to C₅ lower alcohols, ethanol is particularly preferably used as a diluent solvent.

The amount of the diluent solvent added is not particularly limited as long as it can dilute the constituent ingredient to be diluted to the extent allowing for dispensing with a pipette or the like. The diluent solvent is added so that the concentration of the constituent ingredient to be diluted can be, for example, 5 to 80 w/w%, more specifically 25 to 60 w/w%.

### 2. Step (b) of mixing oil and surfactants

In the subsequent step (b), plural test mixtures are prepared by dispensing the ingredients contained in the dilutions and ingredients other than those ingredients into plural test vessels so that the kinds and mixed amounts of the ingredients and the other ingredients added differ among the test vessels.

As described above, oil (O), a hydrophilic surfactant (S), a lipophilic surfactant (CoS), and, if necessary, an optional absorption promoter and auxiliary solvent are usually added to an SEDDS formulation.

The oil (lipid) used here is not particularly limited, and examples thereof can include fatty acid esters specifically including: propylene glycol monocaprate (NIKKOL Sefsol-218, Nikko Chemicals Co., Ltd.; hereinafter, referred to as "Sefsol-218"), propylene glycol dicaprate (NIKKOL Sefsol-228, Nikko Chemicals Co., Ltd.; hereinafter, referred to as "Sefsol-228"), triacetin, fats and oils such as olive oil, sesame oil, soybean oil, corn oil, rape oil, castor oil, coconut oil, and eucalyptus oil; Miglyol 812; triglyceride such as tricaprylin and trilaurin; and polyglycerin fatty acid esters such as tetraglycerin polyricinoleate, hexaglycerin polyricinoleate, condensed polyricinoleate, and tetraglycerin mixed fatty acid esters. Among these oils, examples of substances dispensable without particular dilution with a diluent solvent can include "Sefsol-218", "Sefsol-228", Miglyol 812, and triglyceride such as tricaprylin and trilaurin.

The hydrophilic surfactant (HLB (hydrophile-lipophile balance) of 9.0 or higher) used here is not particularly limited, and examples thereof can include polyoxyethylene lauryl ethers (Laureth 2 (BL-2), Laureth 4.2 (BL-4.2), and Laureth 9 (BL-9)), polyoxyethylene (20) sorbitan monococonut oil fatty acid ester (NIKKOL TL-10, Nikko Chemicals Co., Ltd.; hereinafter, referred to as "Polysorbate 20"), Polysorbate 40, Polysorbate 80, Labrasol, D-α-tocopheryl polyethylene glycol 1000 succinate (Vitamin E TPGS NF, EASTMAN Chemical; hereinafter, referred to as "TPGS NF"), lauroyl polyoxyethylene glycerin (Gelucire 44/14, Gattefosse), polyoxyethylene hydrogenated castor oil 40 (HCO-40), and polyoxyethylene hydrogenated castor oil 60 (HCO-60), polyoxyethylene sorbitan monolaurate, polyoxyethylene sorbitan monopalmitate, and polyoxyethylene sorbitan monooleate.

The lipophilic surfactant (HLB of 0 to less than 9.0) used here is not particularly limited, and examples thereof can include sorbitan monooleate (NIKKOL SO-10V, Nikko Chemicals Co., Ltd.; hereinafter, referred to as "SO-10"), propylene glycol monocaprylate (CAPRYOL 90, Gattefosse; hereinafter, referred to as "Capryol 90"), propylene glycol monolaurate (LAUROGLYCOL 90, Gattefosse; hereinafter, referred to as "Lauroglycol 90"), propylene glycol laurate (LAUROGLYCOL FCC, Gattefosse; hereinafter, referred to as "Lauroglycol FCC"), polyoxyethylene (3) castor oil (NIKKOL CO-3, Nikko Chemicals Co., Ltd.; hereinafter, referred to as "CO-3"), polyoxyethylene (10) castor oil (NIKKOL CO-10, Nikko Chemicals Co., Ltd.; hereinafter, referred to as "CO-10"), decaglyceryl pentaoleate (NIKKOL Decaglyn 5-OV, Nikko Chemicals Co., Ltd.; hereinafter, referred to as "Decaglyn 5-0"), diglyceryl monooleate (NIKKOL DGMO-CV, Nikko Chemicals Co., Ltd.; hereinafter, referred to as "DGMO-C"), hexaglyceryl dioleate (PULUROL OLEIQUE 497, Gattefosse; hereinafter, referred to as "Pulurol oleique 497"), polyoxyethylene glycerin (6) oleate (LABRAFIL M 1944 CS, Gattefosse; hereinafter, referred to as "Labrafil 1944"), and polyoxyethylene glycerin (6) linolenate (LABRAFIL M 2125 CS, Gattefosse; hereinafter, referred to as "Labrafil 2125").

For preparing an SEDDS formulation, for example, an absorption promoter such as sodium salicylate, sodium deoxycholate, sodium myristate, or sodium dodecyl sulfate and an auxiliary solvent such as ethanol, propylene glycol, polyethylene glycol, diethylenetriaminepentaacetic acid, diethanolamine, triethanolamine, ethylenediamine, monoethanolamine, or N,N-dimethylacetamide can be formulated in addition to the above-described ingredients. For example, when propylene glycol or polyethylene glycol is used as an auxiliary solvent, it is preferred to dilute the auxiliary solvent with a diluent solvent.

Other oils, surfactants, and additives that can be used in the present invention can be obtained with reference to documents and reference books known in the art (see e.g., Japanese Pharmacopoeia, 13th Edition; The Japanese Pharmaceutical Codex 1997; Japanese Pharmaceutical Excipients 1998; Japan's Specifications and Standards for Food Additives, 7th Edition; The Japanese Standards of Cosmetic Ingredients, New Edition; The Japanese Cosmetic Ingredients Codex; The Japanese Standards of Quasi-drug Ingredients; United States Pharmacopeia 24; British Pharmacopeia 2000; European Pharmacopeia 2000; and National Formulary 19).

For ingredients that require dilution with a diluent solvent for dispensing among the above-described ingredients for SEDDS formulations, each dilution is prepared on the basis of the method of the step (a), and these dilutions are dispensed into plural test vessels with a dispensing apparatus (tool). Other ingredients for SEDDS formulations dispensable without dilution are dispensed into the plural test vessels with a dispensing apparatus (tool), either directly or after being diluted with a diluent solvent, if necessary. The dispensing order of the ingredients is not particularly limited as long as SEDDS formulations can be prepared. Usually, (1) an active ingredient, (2) oil, (3) a hydrophilic surfactant, and (4) a lipophilic surfactant are dispensed in this order into test vessels. Examples of the dispensing apparatus (tool) include pipettes and LAS pipettes. Examples of the test vessels used here include test tubes and plates that can accommodate therein plural test substances (e.g., 96-well plates and 384-well plates). In the present invention, commercially available LAS would be used preferably, and for example, GENESIS Workstation 200 (manufactured by TECAN) or MultiPROBE II plus (manufactured by Perkin Elmer) can be used. The use of such LAS allows the automated, simultaneous dispensing of many formulations at small amounts of samples. When the ingredients are diluted with the same kinds of diluent solvents (e.g., lower alcohols), those plural ingredients dispensed can be mixed more easily. Moreover, LAS allows low-volume dispensing and can therefore reduce the amounts of drugs used to small amounts.

The ingredients are dispensed at an appropriately adjusted different ratio into test vessels. For example, ranges such as S:CoS=10:0 to 6:4 and (S+CoS):O=10:0 to 6:4 can be selected as such a ratio. After the addition of these ingredients, the mixture is stirred for an appropriate time by appropriate means (e.g., at 500 to 2000 rpm for 20 seconds to 1 minute). The present invention can also provide a screening kit for an SEDDS formulation comprising, for example, the thus-prepared dilution containing each constituent ingredient.

### 3. Step (c) of removing diluent solvent

In the method of the present invention, the diluent solvent added in the step (a) is usually removed. Such removal of the diluent solvent is not particularly limited and is usually performed by drying under reduced pressure. When ethanol is used as a diluent solvent, the ethanol may be removed by drying, for example, at room temperature to 40°C under reduced pressure for approximately 4 to 10 hours (e.g., overnight). In this way, the diluent solvent can be removed from each test mixture accommodated in each test vessel to thereby prepare plural candidate formulations of emulsion preparations. The candidate formulations of emulsion preparations thus obtained are evaluated in the subsequent step for whether they are appropriate formulations as the self-emulsifying preparation.

### 4. Step (d) of adding water

The self-emulsifying preparation is, as described above, a preparation that is obtained by uniformly mixing the above-described components in a state free from water to prepare a solution or dispersion. The self-emulsifying preparation is designed so that this preparation after administration forms a fine emulsion upon dissolution and dispersion in water, gastric juice, or mixtures thereof in the gastroentestinal tract. Thus, to evaluate whether the candidate formulations of emulsion preparations thus prepared are appropriate, water or a test solution is initially added thereto to form emulsions. In this context, the "test solution" means a liquid capable of bringing about the self-emulsification of SEEDS. The test solution used is, for example, artificial gastric juice (e.g., Test Solution 1 (JP1) or Test Solution 2 (JP2) described in Japanese Pharmacopoeia, 14th Edition) or a solution of the artificial gastric juice diluted with water. The amount of water or the test solution added is appropriately changed according to the kind and amount of each ingredient. For example, water or the test solution is added in an amount 5 to 20 times the amount of the test mixture to each test vessel. After the addition of water or the test solution, the mixture is usually stirred to form an emulsion. The stirring is conducted, for example, but not limited to, at 500 to 2000 rpm for approximately 20 seconds to 1 minute.

### 5. Step (e) of evaluating formulations of emulsion preparations obtained

In the present invention, the properties of the emulsions thus formed are subsequently evaluated. Indexes for indicating the physical properties of an emulsion include: a particle size; and the separation of an emulsion that indicates physical stability. Therefore, these properties are usually evaluated. Conventional methods known in the art can be used as methods for evaluating such properties. For example, dynamic light scattering (hereinafter, referred to as "DLS") can be used as a method for measuring a particle size. Alternatively, a method for evaluating, with a turbidimeter, turbidity as a parameter that reflects a particle size can also be used.

However, the present inventor has found that the particle size of an emulsion formed by SEDDS can be evaluated quickly by turbidity measurement using an absorption spectrometer for microplates. The present inventor has also found that the separation stability of SEDDS can be evaluated by a turbidity change.

Thus, in the preferable aspect of the present invention,
(e-1) a method for evaluating the particle sizes of the emulsions by turbidity measurement;
(e-2) a method for evaluating the separation stability of the emulsions by the measurement of a turbidity change between before and after storage treatment; and/or
(e-3) a method for evaluating the separation stability of the emulsions by the measurement of a turbidity change between before and after centrifugation treatment can be used as a method for evaluating the emulsions.

According to a more preferable aspect of the present invention, two or more of the evaluation methods (e-1) to (e-3) are combined to perform evaluation, and on the basis of the results, the optimum formulation of the preparation is determined.

Hereinafter, the evaluation methods (e-1) to (e-3) are described.

### e-1: method for evaluating particle sizes by turbidity measurement

The appearance of an SEDDS aqueous solution is generally known to vary depending on the particle size of its emulsion. The appearances can broadly be divided into three states: (1) a clear appearance resulting form the formation of an emulsion of approximately 150 nm or smaller in particle size (microemulsion; hereinafter referred to as "ME"; turbidity in an amount of 200 µl/well is less than 0.3); (2) a clear, but whitish appearance resulting from a particle size of an emulsion larger than that of ME or from the formation of this emulsion having a larger particle size together with ME (whitish microemulsion; hereinafter, referred to as "WME"; turbidity in an amount of 200 µl/well is 0.3 to 1); and (3) an opaque, white appearance resulting from the formation of an emulsion having a particle size on a 1 µm order (macroemulsion; hereinafter, referred to as "MacE"; turbidity in an amount of 200 µl/well exceeds 1). As described above, the appearance of an emulsion greatly varies depending on its particle size. Therefore, particle sizes can be evaluated easily by turbidity measurement. Alternatively, the use of an absorption spectrometer for microplates (e.g., SpectraMax 190, Molecular Devices Corp.) allows measurement using low volumes of samples and therefore allows quick evaluation at low cost. Thus, such a method for evaluating particle sizes according to the present invention can quickly evaluate the particle sizes of many SEDDS formulations at low cost with small amounts of dugs used.

### e-2 and e-3: Methods for evaluating separation stability by turbidity change

In the methods for evaluating separation stability by a turbidity change according to the present invention, the separation stability of the emulsions is evaluated by the measurement of a turbidity change between before and after storage treatment or between before and after centrifugation treatment of the emulsions. For example, the emulsions thus prepared are stored for 6 to 72 hours inclusive under conditions of 1 to 40°C inclusive. The presence or absence of the separation of SEDDS can be evaluated by a turbidity change between before and after the storage.

Alternatively, to conduct evaluation in a shorter time under more strict conditions, the prepared emulsions can be subjected to centrifugation treatment. In this case, for example, the emulsions are centrifuged under conditions of 1500 to 2000 rpm, and a turbidity change between before and after the centrifugation is measured. In this case, the time-dependent aggregation of particles in an emulsion increases the particle sizes. The particles with the increased sizes are separated by the centrifugation treatment, and this emulsion comprising the separated particles can be evaluated accurately and quickly on the basis of its turbidity. The separated states are divided into (1) separation into a cream layer and a clear layer and (2) separation into a clear layer and a clear layer. Such separations can also be evaluated on the basis of turbidity. According to circumstances, the precipitation of a drug or the generation of insoluble matters attributed to incompatibility between the formulated ingredients can also be detected. As described above, turbidity has the advantage of being capable of being quickly evaluated at low cost with low volumes of samples.

The method of the present invention comprising the combined steps (a) to (e) can complete HTFS using the improved convenience and quickness of LAS in SEDDS formulation design that requires, due to the discontinuously varying formation state and stability of an emulsion, screening by trial and error in which a large number of formulations are prepared and evaluated. Moreover, the method of the present invention can quickly evaluate the separation stability of many formulations at low cost with small amounts of drugs used.

### Examples and Comparative Examples

Hereinafter, the present invention will be described more specifically with reference to Examples of the present invention. However, the present invention is not intended to be limited to these Examples, and various changes may be made therein without departing from the technical scopes of the present invention.

### Comparative Example 1: Sample preparation without use of diluent solvent

To prepare an SEDDS formulation, a surfactant used was polyoxyethylene (40) hydrogenated castor oil (NIKKOL HCO-40 (for medical purposes), Nikko Chemicals Co., Ltd.; hereinafter, referred to as "HCO-40"), and oil used was medium-chain fatty acid glyceride (ODO-C, The Nisshin OilliO Group, Ltd.; hereinafter, referred to as "MCT"). The formulation is described in Table 1.

**Table 1**

| Ingredient | Volume (µL) |
|---|---|
| HCO-40 | 300 |
| MCT | 150 |
| Water | 2400 |
| Total | 2850 |

HCO-40 was viscous even after heat melting and as such, could not be dispensed with a pipette. Thus, a weight equal to 300 µL was measured after heat melting. To this aliquot, 150 µL of MCT was dispensed and then stirred. Uniform mixing was difficult due to the viscosity of HCO-40. Thus, mixing was achieved with a spatula. Finally, 2400 µL of water was added thereto to prepare Sample 1.

### Comparative Example 2: Sample preparation with water as diluent solvent

After heat melting, HCO-40 was dissolved at a concentration of 50 w/w% in water. However, this solution was made into an exceedingly viscous gel and as such, could not be dispensed.

Thus, supposing that dispensing with a pipette could be achieved, a weight equal to 300 µL of HCO-40 was directly measured into a vial. To the vial, 300 µL of water was dispensed and then stirred. Subsequently, 150 µL of MCT was dispensed thereto. However, uniform stirring was difficult due to the gelation of the HCO-40 aqueous solution.

Thus, the stirring was forcedly achieved with a spatula. Then, the mixture was dried for 12 hours in a vacuum drier. To the mixture, 2400 µL of water was added to prepare Sample 2.

### Example 1: Sample preparation with ethanol as diluent solvent

After heat melting, HCO-40 was dissolved at a concentration of 50 w/w% in ethanol. A 600 µL aliquot (300 µL in terms of HCO-40) of this solution and 150 µL of MCT were dispensed into a vial with a pipette. The mixture was stirred to make it uniform. Then, the mixture was dried for 12 hours in a vacuum drier. To the mixture, 2400 µL of water was added to prepare Sample 3. (Test Example 1) Evaluation of influence of diluent solvent on complexity of sample preparation

As described in Comparative Example 1, HCO-40 could not be dispensed with a pipette without use of a diluent solvent. Even in Comparative Example 2 with water as a diluent solvent, the dispensing of HCO-40 with a pipette was difficult. Moreover, in Comparative Examples 1 and 2, the uniform mixing of HCO-40 with MCT was difficult. However, as described in Example 1 of the present invention, the dispensing of HCO-40 with a pipette could be achieved with ethanol as a diluent solvent, and this dispensing could be performed easily and quickly. Moreover, in Example 1, HCO-40 could be mixed with MCT easily and quickly.

### (Test Example 2) Evaluation of influence of diluent solvent on emulsion (1)

The appearances of the Samples 1 to 3 of Comparative Examples 1 and 2 and Example 1 of the present invention were visually observed. These three samples were dispensed in an amount of 200 µL each into a microplate. Their turbidities were measured at 650 nm with an absorption spectrometer (SpectraMax 190, Molecular Devices Corp.). The Sample 1 obtained without use of a diluent solvent was used as a control. The results are shown in Table 2A.

**Table 2A**

| | Sample 1 | Sample 2 | Sample 3 |
|---|---|---|---|
| Appearance | Clear | Clear whitish | Clear |
| Type | ME | WME | ME |
| Turbidity | 0.04 | 0.23 | 0.02 |
| n=3 | | | |

In this Table, ME (microemulsion) denotes a clear appearance resulting from the formation of an emulsion of approximately 100 nm or smaller in particle size. MWE (whitish microemulsion) denotes a clear, but whitish appearance resulting from a particle size of an emulsion larger than that of ME or from the formation of this emulsion having a larger particle size together with ME. Alternatively, MacE (macroemulsion) denotes an opaque, white appearance resulting from the formation of an emulsion having a particle size on a 1 µm order.

The Sample 2 obtained with water as a diluent solvent had a clear, but whitish appearance and was therefore WME, when compared with the Sample 1 (clear, ME) obtained without use of a diluent solvent. However, the Sample 3 obtained with ethanol as a diluent solvent was clear and was ME, as with the Sample 1. Moreover, the Sample 2 had turbidity (0.23) greatly differing from the turbidity (0.04) of the Sample 1, indicating an increased particle size. By contrast, the Sample 3 had turbidity (0.02) almost equal to that of the Sample 1. Thus, ethanol was demonstrated to be more preferable than water as a diluent solvent that can correctly reflect the original physical property of an emulsion.

### Comparative Example 3

A sample obtained with water as a diluent solvent, as with the Sample 2, but without drying treatment was prepared as follows: after the heat melting of HCO-40, a weight equal to 300 µL of HCO-40 was directly measured into a vial. To the vial, 300 µL of water was dispensed and then stirred. Subsequently, 150 µL of MCT was dispensed thereto. Mixing was achieved with a spatula. To the mixture, 2400 µL of water was added to prepare Sample 4.

### Example 2

A sample obtained with ethanol as a diluent solvent, as with the Sample 3, but without drying treatment was prepared as follows: after heat melting, HCO-40 was dissolved at a concentration of 50 w/w% in ethanol. A 600 µL aliquot (300 µL in terms of HCO-40) of this solution and 150 µL of MCT were dispensed into a vial with a pipette. The mixture was stirred to make it uniform. To the mixture, 2400 µL of water was added to prepare Sample 5.

### Test Example 3: Evaluation of influence of residual solvent on emulsion

To clearly show the influence of the residual solvents, the Samples 4 and 5 were prepared without drying treatment. The appearances of the Samples 1, 4, and 5 of Comparative Examples 1 and 3 and Example 2 of the present invention were visually observed. These three samples were dispensed in an amount of 200 µL each into a microplate. Their turbidities were measured at 650 nm with an absorption spectrometer (SpectraMax 190, Molecular Devices Corp.). The Sample 1 obtained without use of a diluent solvent was used as a control. The results are shown in Table 2B.

**Table 2B**

| | Sample 1 | Sample 4 | Sample 5 |
|---|---|---|---|
| Appearance | Clear | White | Clear |
| Type | ME | MacE | ME |
| Turbidity | 0.05 | 2.32 | 0.02 |
| n=3 | | | |

The Sample 4 obtained with water had a white appearance and formed MacE having turbidity of 2.32, which greatly differed from the emulsion of the Sample 1. On the other hand, the Sample 5 obtained with ethanol had a clear appearance and formed ME, as with the Sample 1. Moreover, the Sample 5 had turbidity of 0.05 almost equal to that of the Sample 1. Thus, ethanol was demonstrated to be more preferable than water as a diluent solvent that can correctly reflect the original physical property of an emulsion.

### Example 3: Dispensing precision

Surfactants diluted at a concentration of 50 w/w% with ethanol were used to evaluate the dispensing precision of LAS (GENESIS Workstation 200, TECAN). The set value of the dispensed volume was 200 µL.

The surfactants used were polyoxyethylene (2) lauryl ether (NIKKOL BL-2, Nikko Chemicals Co., Ltd.; hereinafter, referred to as "BL-2"); polyoxyethylene (4.2) lauryl ether (NIKKOL BL-4.2, Nikko Chemicals Co., Ltd.; hereinafter, referred to as "BL-4.2"); polyoxyethylene (9) lauryl ether (NIKKOL BL-9EX, Nikko Chemicals Co., Ltd.; hereinafter, referred to as "BL-9"); polyoxyethylene (20) sorbitan monopalmitate (NIKKOL TP-10V, Nikko Chemicals Co., Ltd.; hereinafter, referred to as "Polysorbate 40"); polyoxyethylene (20) sorbitan monooleate (NIKKOL TO-10MV, Nikko Chemicals Co., Ltd.; hereinafter, referred to as "Polysorbate 80"); polyoxyethylene (8) glycerin caprylate-caprate (LABRASOL, Gattefosse; hereinafter, referred to as "Labrasol"); HCO-40; and polyoxyethylene (60) hydrogenated castor oil (NIKKOL HCO-60 (for medical purposes), Nikko Chemicals Co., Ltd.; hereinafter, referred to as "HCO-60").

The dispensed volumes were calculated from the weights and densities of the dispensed surfactant solutions. The results are shown in Table 3.

**Table 3**

| Surfactant | RSD |
|---|---|
| BL-2 | 0.0046 |
| BL-4.2 | 0.0153 |
| BL-9 | 0.0257 |
| Polysorbate 40 | 0.0034 |
| Polysorbate 80 | 0.0189 |
| Labrasol | 0.0072 |
| HCO-40 | 0.0029 |
| n=3 | |

As can be seen from Table 3, all the solutions showed RSD (relative standard deviation) of 0.03 or lower and were demonstrated to be dispensed with good precision. Thus, the use of ethanol as a diluent solvent allowed for the dispensing of a variety of surfactants with a pipette.

### Example 4: Execution of HTFS

Oil and surfactants shown below were dispensed into a 96-well plate with LAS (GENESIS Workstation 200, TECAN). The surfactants were used in the form of 50 v/v% ethanol solution.

A hydrophilic surfactant (hereinafter, referred to as "S") and a lipophilic surfactant (hereinafter, referred to as "CoS") were mixed at a ratio of S:CoS=10:0, 9:1, 8:2, 7:3, or 6:4. The mixed surfactants were further mixed with oil (hereinafter, referred to as "O") at a ratio of (S+CoS):0=10:0, 9:1, 8:2, 7:3, or 6:4 (mixtures containing HCO-60 were prepared at ratios up to 5:5, 4:6, 3:7, 2:8, and 1:9). The oil and the surfactants were dispensed in a total amount of 50 µL in terms of SEDDS. Nilvadipine (Kongo Yakuhin Co., Ltd.; hereinafter, referred to as "Nil") was used as a model compound.

The drug cannot be dispensed directly in a powder form. Therefore, the drug was prepared into 5 mg/mL ethanol solution and added at a Nil concentration of 4 mg/mL per mL of SEDDS. Each mixture of the drug, the oil, and the hydrophilic and lipophilic surfactants was stirred to make it uniform. Then, the mixtures were vacuum-dried overnight (for 12 hours or longer) to evaporate ethanol as a diluent solvent. After the drying, 450 µL of distilled water was added to each of the mixtures and stirred to prepare samples.

### (Oil used)

The oil used was propylene glycol monocaprate (NIKKOL Sefsol-218, Nikko Chemicals Co., Ltd.; hereinafter, referred to as "Sefsol-218").

### (Hydrophilic surfactant used)

The hydrophilic surfactants used were BL-2, BL-4.2, BL-9, polyoxyethylene (20) sorbitan monococonut oil fatty acid ester (NIKKOL TL-10, Nikko Chemicals Co., Ltd.; hereinafter, referred to as "Polysorbate 20"), Polysorbate 40, Polysorbate 80, Labrasol, D-α-tocopheryl polyethylene glycol 1000 succinate (Vitamin E TPGS NF, EASTMAN Chemical; hereinafter, referred to as "TPGS NF"), lauroyl polyoxyethylene glycerin (Gelucire 44/14, Gattefosse), HCO-40, and HCO-60.

### (Lipophilic surfactant used)

The lipophilic surfactants used were sorbitan monooleate (NIKKOL SO-10V, Nikko Chemicals Co., Ltd.; hereinafter, referred to as "SO-10"), propylene glycol monocaprylate (CAPRYOL 90, Gattefosse; hereinafter, referred to as "Capryol 90"), propylene glycol monolaurate (LAUROGLYCOL 90, Gattefosse; hereinafter, referred to as "Lauroglycol 90"), propylene glycol laurate (LAUROGLYCOL FCC, Gattefosse; hereinafter, referred to as "Lauroglycol FCC"), polyoxyethylene (3) castor oil (NIKKOL CO-3, Nikko Chemicals Co., Ltd.; hereinafter, referred to as "CO-3"), polyoxyethylene (10) castor oil (NIKKOL CO-10, Nikko Chemicals Co., Ltd.; hereinafter, referred to as "CO-10"), decaglyceryl pentaoleate (NIKKOL Decaglyn 5-OV, Nikko Chemicals Co., Ltd.; hereinafter, referred to as "Decaglyn 5-O"), diglyceryl monooleate (NIKKOL DGMO-CV, Nikko Chemicals Co., Ltd.; hereinafter, referred to as "DGMO-C"), hexaglyceryl dioleate (PULUROL OLEIQUE 497, Gattefosse; hereinafter, referred to as "Pulurol oleique 497"), polyoxyethylene glycerin (6) oleate (LABRAFIL M 1944 CS, Gattefosse; hereinafter, referred to as "Labrafil 1944"), and polyoxyethylene glycerin (6) linolenate (LABRAFIL M 2125 CS, Gattefosse; hereinafter, referred to as "Labrafil 2125").

### (Measurement of turbidity of SEDDS)

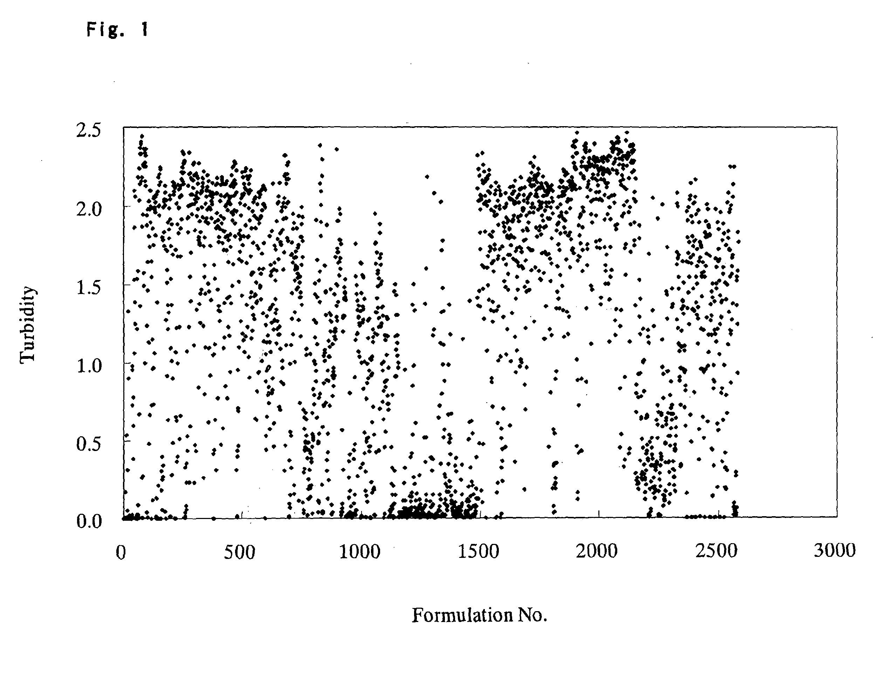

The samples were dispensed in an amount of 200 µL each into a microplate. Their turbidities were measured at 650 nm with an absorption spectrometer (SpectraMax 190, Molecular Devices Corp.) for type determination depending on particle sizes. The results have been shown in Figure 1. The formulation having low turbidity means that the emulsion has a low particle size. The formulation having turbidity of 0.3 or lower is ME.

### (Measurement of turbidity change after centrifugation)

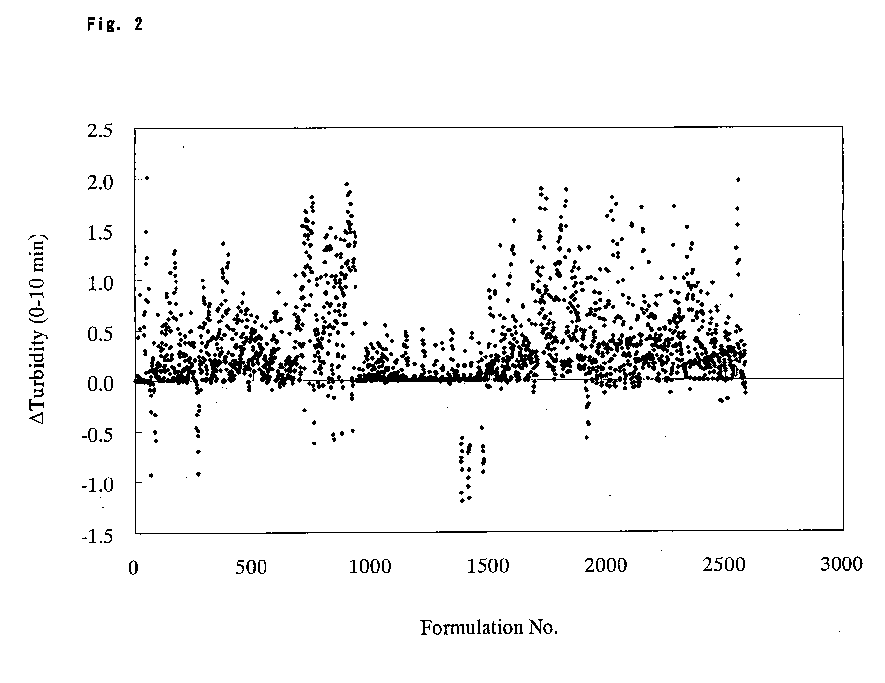

Subsequently, the plate was centrifuged at 500 g for 10 minutes with a centrifuge (LC-120, Tomy Seiko Co., Ltd.). A turbidity (650 nm) change between before and after the centrifugation was determined. The results have been shown in Figure 2. The turbidity change occurs depending on physical changes such as the separation of SEDDS into oil and aqueous phases and the precipitation of a drug. Formulations having a turbidity change of - 0.1 to 0.1 were selected as those having excellent physical stability.

### (Turbidity change after storage at 25°C for 48 hours)

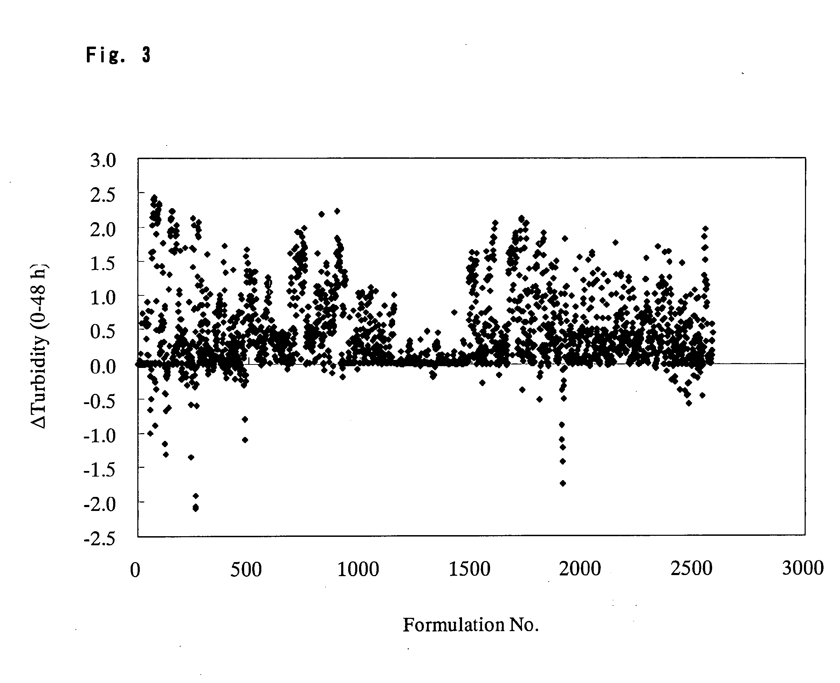

A 96-well plate into which the samples were dispensed in an amount of 200 µL each in the same way as above was stored at 25°C for 48 hours. A turbidity (650 nm) change between before and after the storage was measured. The results have been shown in Figure 3. Formulations having a turbidity change of -0.1 to 0.1 were selected as those having excellent physical stability.

From these HTFS results, formulations shown in Table 4 below could be designed as formulations in an ME form having excellent physical stability in centrifugation and further having excellent physical stability at 25°C.

**Table 4**

| S | CoS | S:CoS | (S+CoS):O |
|---|---|---|---|
| Polysorbate 20 | Labrafil1944 | 9:1 | 8:2 |
| Polysorbate 40 | Capryol90 | 9:1 | 9:1 |
| Polysorbate 80 | CO-10 | 9:1 | 9:1 |
| Labrasol | SO-10 | 9:1 | 9:1 |
| HCO-40 | CO-3 | 6:4 | 6:4 |
| HCO-60 | DGMO-C | 7:3 | 5:5 |
| BL-9 | CO-10 | 6:4 | 9:1 |

### Industrial Applicability

The present invention can provide an HTFS system for quickly preparing and evaluating, at low cost, an SEDDS formulation containing a poorly soluble active ingredient and so on. Thus, the present invention has the advantage of being capable of quickly developing the optimum SEDDS formulation.

## Claims

1. A method for formulation designing of a self-emulsifying preparation of a poorly water-soluble drug, comprising the steps of: (a) dissolving, in a diluent solvent, each of the poorly water-soluble drug and ingredients of the preparation in a viscous liquid state or in a semi-solid or solid state at room temperature to prepare each dilution containing the ingredient; and (b) if additional ingredients of the preparation that are dispensable with a dispensing apparatus at room temperature are required, using each of the dispensable ingredients as it is without dissolution in a diluent solvent or dissolving each of the dispensable ingredients in a diluent solvent to prepare each dilution containing the dispensable ingredient, and preparing plural test mixtures by dispensing all these ingredients into plural test vessels so that the kinds and mixed amounts of the ingredients added differ among the test vessels.

2. The method according to claim 1, further comprising the steps of: (c) removing the diluent solvent from the plural test mixtures obtained; (d) adding water or a test solution to the plural test mixtures obtained; and (e) evaluating the properties of emulsions formed from the plural test mixtures thus obtained.

3. The method according to claim 1 or 2, wherein the diluent solvent is alcohols.

4. The method according to claim 3, wherein the diluent solvent is an alcohol having 2 to 5 carbon atoms.

5. The method according to claim 4, wherein the diluent solvent is ethanol, n-propanol, isopropanol, or a mixture thereof.

6. The method according to any of claims 2 to 5, wherein the step (e) of evaluating the properties of emulsions formed from the test mixtures comprises the step of evaluating the particle sizes of the emulsions by turbidity measurement.

7. The method according to any of claims 2 to 6, wherein the step (e) of evaluating the properties of emulsions formed from the test mixtures comprises the step of evaluating the separation stability of the emulsions by the measurement of a turbidity change between before and after storage treatment.

8. The method according to any of claims 2 to 7, wherein the step (e) of evaluating the properties of emulsions formed from the test mixtures comprises the step of evaluating the separation stability of the emulsions by the measurement of a turbidity change between before and after centrifugation treatment.

9. The method according to any of claims 2 to 8, further comprising the step of determining the optimum formulation of the self-emulsifying preparation on the basis of evaluation results obtained in the step of evaluating the properties of emulsions formed from the text mixtures.
